# EUROPEAN PATENT APPLICATION

(11) **EP 3 457 160 A1**
(43) Date of publication of application: **20.03.2019**
(21) Application number: 17191004.5
(22) Date of filing: 14.09.2017
(51) Int. Cl.: G01R 33/561, G01R 33/58

(54) **PARALLEL MAGNETIC RESONANCE IMAGING WITH ARCHIVED COIL SENSITIVITY MAPS**

(71) Applicant: Koninklijke Philips N.V., 5656 AE Eindhoven (NL)
(72) Inventor: VAN DEN BRINK, Johan Samuel, 5656 AE Eindhoven (NL)
(74) Representative: de Haan, Poul Erik

(57) **Abstract**

The invention provides for a magnetic resonance imaging system (100, 300, 500) comprising a radio-frequency system (114, 116) comprising multiple coil elements (114) for acquiring imaging magnetic resonance data (166) from a subject (118). The magnetic resonance imaging system further comprises a memory (150) for storing machine executable instructions (160). The memory further stores imaging pulse sequence commands (164). The imaging pulse sequence commands are configured for controlling the magnetic resonance imaging system to acquire the imaging magnetic resonance data according to a chosen parallel magnetic resonance imaging protocol. The magnetic resonance imaging system further comprises a processor (144) for controlling the magnetic resonance imaging system. Execution of the machine executable instructions causes the processor to: control (200) the magnetic resonance imaging system to acquire the imaging magnetic resonance data using the pulse sequence commands; and reconstruct (202) an imaging magnetic resonance image (168) from the imaging magnetic resonance data according to the chosen parallel magnetic resonance imaging protocol. The imaging magnetic resonance image is reconstructed by maximizing consistency between the imaging magnetic resonance data, the imaging magnetic resonance image, and an imaging coil sensitivity map (162). After reconstructing the imaging magnetic resonance image, the processor stores (202) the imaging coil sensitivity map in the memory.

## Description

### TECHNICAL FIELD

The invention relates to magnetic resonance imaging, in particular to parallel imaging techniques in magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field.

One method of spatially encoding is to use magnetic field gradient coils. Typically there are three coils which are used to generate three different gradient magnetic fields in three different orthogonal directions.

During an MRI scan, Radio Frequency (RF) pulses generated by one or more transmitter coils cause a called B1 field. Additionally applied gradient fields and the B1 field do cause perturbations to the effective local magnetic field. RF signals are then emitted by the nuclear spins and detected by one or more receiver coils. Data can be acquired separately by the individual receiver coils. Images reconstructed from data for each of the individual receiver coils can be combined into a single image or image data using SENSitivity Encoding (SENSE) magnetic resonance imaging techniques as discussed in the Journal article Pruessmann et. al., (1999), "SENSE: Sensitivity encoding for fast MRI," Magn. Reson. Med., 42: 952-962, doi:10.1002/(SICI)1522-2594(199911)42:5<952::AID-MRM16>3.0.CO;2-S.

United States patent application publication US 2017/0237865 discloses Magnetic resonance (MR) imaging performed in cooperation with an MR scanner that uses a method comprising: (i) acquiring sensitivity maps for a plurality of radio frequency coils using a MR pre scan performed by the MR scanner; (ii) acquiring an MR imaging data set using the plurality of radio frequency coils and the MR scanner; and (iii) reconstructing the MR imaging data set using partially parallel image reconstruction employing the sensitivity maps and a correction for subject motion between the acquiring (i) and the acquiring (ii).

### SUMMARY OF THE INVENTION

The invention provides for a magnetic resonance imaging system, a computer program product and a method in the independent claims. Embodiments are given in the dependent claims.

Parallel magnetic resonance imaging protocols are magnetic resonance imaging protocols that use a coil sensitivity map or matrix in order to reconstruct a magnetic resonance image. Herein, the terms parallel magnetic resonance imaging protocol and parallel imaging protocol are used interchangeably. For some protocols, the coil sensitivity map is separately measured before or after the acquisition of the magnetic resonance data. In other parallel imaging protocols the coil sensitivity maps are determined by maximizing a consistency between the reconstructed image, the magnetic resonance data, and the coil sensitivity maps. An example of a parallel imaging protocol is a SENSE or SENSE like magnetic resonance imaging protocol.

Embodiments of the invention may be, in some examples, used to provide an imaging coil sensitivity map that has been optimized by maximizing the consistency between the above mentioned the reconstructed image, the magnetic resonance data, and the coil sensitivity maps. Embodiments may also store this optimized coil sensitivity map. This may for example be useful for future use with other parallel imaging protocols.

In one aspect, the invention provides for a magnetic resonance imaging system comprising a radio-frequency system comprising multiple coil elements for acquiring imaging magnetic resonance data from a subject. Imaging magnetic resonance data is magnetic resonance data. The term imaging before imaging magnetic resonance data is a label to indicate specific magnetic resonance data. A radio-frequency system may comprise a transmitter, receiver and/or transceiver and multiple coil elements. The multiple coil elements may be configured for sending and/or receiving radio-frequency signals on independent channels.

The magnetic resonance imaging system further comprises a memory for storing machine-executable instructions. The memory further stores imaging pulse sequence commands. The imaging pulse sequence commands are configured for controlling the magnetic resonance imaging system to acquire the imaging magnetic resonance data according to an imaging parallel magnetic resonance imaging protocol. The imaging pulse sequence commands are pulse sequencing commands. The term imaging before imaging pulse sequence commands is a label to indicate specific pulse sequence commands. A chosen parallel magnetic resonance imaging protocol is a parallel imaging protocol. A coil sensitivity map as used herein may also be referred to as a coil sensitivity matrix.

There are several different ways of driving or acquiring coil sensitivity maps. In one method, low resolution images are acquired for each of the multiple coil elements and then are compared to a reference image such as a full body coil image. These coil sensitivities map or matrix can then be used to reconstruct the image for a parallel imaging protocol. The coil sensitivity maps could be measured using the subject being imaged or they could have been previously acquired using phantoms. For example, during system installation, manufacturing, or service.
There are also other types of parallel imaging protocols that acquire the data necessary for determining the coil sensitivities during the course of acquiring the data as was mentioned above. Such methods are also denoted as auto-calibration parallel imaging protocols.

The magnetic resonance imaging system further comprises a processor for controlling the magnetic resonance imaging system. Execution of the machine executable instructions further cause the processor to control the magnetic resonance imaging system to acquire the imaging magnetic resonance data using the pulse sequence commands. Execution of the machine executable instructions further cause the processor to reconstruct an imaging magnetic resonance image from the imaging magnetic resonance data according to the chosen parallel magnetic resonance imaging protocol. The imaging magnetic resonance image is reconstructed by maximizing consistency between the imaging magnetic resonance data, the imaging magnetic resonance image, and an imaging coil sensitivity map. The processor then stores the resulting imaging coil sensitivity map in the memory for future use. This may be advantageous because the imaging coil sensitivity map may be more accurate than a coil sensitivity map that has been measured prior to the parallel imaging protocol. This is particularly beneficial when an iterative reconstruction is used to generate the imaging coil sensitivity map which is self-consistent.

In a specific example, the imaging magnetic resonance image is reconstructed using an iterative least squares algorithm. In one variant, the iterative least squares algorithm is configured for optimizing either a preexisting or archived coils sensitivity map into the imaging coil sensitivity map during reconstruction of the imaging magnetic resonance image. In another variant, the imaging coil sensitivity map is determined wholly through the iterative process, wherein the imaging magnetic resonance image is reconstructed by iteratively maximizing consistency between the imaging magnetic resonance data, the imaging magnetic resonance image, and an imaging coil sensitivity map.

In another embodiment, execution of the machine executable instructions further cause the processor to control the magnetic resonance imaging system to acquire further magnetic resonance data using further pulse sequence commands according to a further parallel magnetic resonance imaging protocol. A further parallel magnetic resonance imaging protocol is a parallel magnetic resonance imaging protocol. The term "further" is a label to differentiate it from the chosen parallel magnetic resonance imaging protocol. In some examples, the further parallel magnetic resonance imaging protocol is identical with the chosen parallel magnetic resonance imaging protocol, however there is no requirement for this to be true. The further parallel magnetic resonance imaging protocol and the chosen parallel magnetic resonance imaging protocol can be different.

Execution of the machine executable instructions further cause the processor to reconstruct a further magnetic resonance image from the further magnetic resonance data using the imaging coil sensitivity map according to the further parallel magnetic resonance imaging protocol. This embodiment may be beneficial because the imaging coil sensitivity map, which is self consistent, may be used at least partially for the reconstruction of the further magnetic resonance image.

In another embodiment, the imaging magnetic resonance image is reconstructed using a fast channel combination method that generates coil sensitivity data for virtual coil elements. Execution of the machine executable instructions causes the processor to store the coil sensitivity data for the virtual coil elements and metadata descriptive of inclusion of the coil sensitivity data in the memory. This embodiment may be beneficial because it may enable reuse of the virtual coil elements and their coil sensitivity data.

In another embodiment, the chosen parallel imaging magnetic resonance imaging protocol is an auto calibration parallel imaging protocol.

In another embodiment, the autocalibrating parallel imaging protocol is a GRAPPA type magnetic resonance imaging protocol.

In another embodiment, the autocalibrating parallel imaging protocol is a eSPIRiT type magnetic resonance imaging protocol.

In another embodiment, the memory further contains calibration pulse sequence commands for acquiring coil sensitivity magnetic resonance data according to a coil sensitivity calibration magnetic resonance imaging protocol, wherein execution of the machine executable instructions further causes the processor to control the magnetic resonance imaging system with the calibration pulse sequence commands to acquire the coil sensitivity magnetic resonance data. Execution of the machine executable instructions further cause the processor to reconstruct a measured coil sensitivity map from the coil sensitivity magnetic resonance data. The imaging coil sensitivity map is derived from the measured coil sensitivity map.

In another embodiment, the memory further contains an archived coil sensitivity map descriptive of coil sensitivities of the multiple coil elements. The imaging coil sensitivity map is derived from the archived coil sensitivity map.

In another embodiment, execution of the machine executable instructions further causes the processor to receive an image quality indicator descriptive of artifacts in the imaging magnetic resonance image. The imaging coil sensitivity map is stored in the memory only if the image quality indicator is above a chosen threshold. The processor may access the memory and retrieve the archived coil sensitivity map during the reconstruction of the imaging magnetic resonance image.

In another embodiment, the memory further contains a database. A database as used herein encompasses a program, data or file source that may be useful for returning data in response to a query. The database for example may be configured for searching for specific data or even data which is closest to the search criteria for the elements in the database. For example the database could be an SQL type database where specific data has responded to particular queries. In other examples the database may use a clustering type algorithm to determine the closest elements or elements to return. The database comprises entries from multiple coil sensitivity maps. The database comprises a meta data entry for each of the multiple coil sensitivity maps. The database is configured for searching the meta data entry for each of the coil sensitivity maps. The meta data may for example be descriptive of the acquisition conditions of when the multiple coil sensitivity maps were acquired.

Execution of the machine executable instructions further comprises receiving one or more selection criteria descriptive of the acquisition of the imaging magnetic resonance data. The receiving of the one or more selection criteria could for example be received from a display or user interface. In other examples the one or more selection criteria may be received automatically from a magnetic resonance imaging system for example by using specific automatically identified coil element positions and/or scan conditions. Execution of the machine executable instructions further causes the processor to query the database with the one or more selection criteria to retrieve the archived coil sensitivity map. The database is configured for selecting the archived coil sensitivity map by comparing the selection criteria to the meta data. This embodiment may be beneficial because it may provide for a means of reusing coil sensitivity maps that have been previously determined for a magnetic resonance imaging system.

In another embodiment; the meta data and/or the one or more selection criteria comprise any one of the following: a field of view for magnetic resonance data, an off-center condition of where the magnetic resonance data is acquired, a subject support position for a subject support of the magnetic resonance imaging system, a coil identifier or serial number for a magnetic resonance imaging coil that comprises the multiple coil elements, a radio-frequency system identifier or serial number for the radio-frequency system, a subject weight of the subject, a subject age of the subject, a subject volume of the subject, a selected anatomical profile, a slice selection for the magnetic resonance data, a slice orientation for the imaging magnetic resonance data, a magnetic resonance imaging scan protocol type for the imaging parallel magnetic resonance imaging protocol, one or more antenna element orientations or positions for the multiple coil elements or the virtual coil elements configuration, a magnetic resonance imaging system identifier or serial number for the magnetic resonance imaging system, a subject identifier, a subject name, and combinations thereof. The selection from one or more of the above for the meta data and/or the one or more selection criteria may have the benefit that it provides a means for grouping prior acquired coil sensitivity maps and reusing them in a new magnetic resonance imaging scan that is done according to an imaging parallel magnetic resonance imaging protocol.

In another embodiment, execution of the machine executable instructions further cause the processor to receive multiple archived coil sensitivity maps from the database. Execution of the machine executable instructions further cause the processor to reconstruct a set of imaging magnetic resonance images from the imaging magnetic resonance data using each of the archived coil sensitivity maps according to the imaging parallel magnetic resonance imaging protocol. Execution of the machine executable instructions further cause the processor to select the imaging magnetic resonance image from the set of imaging magnetic resonance images. In this example more than one coil sensitivity map is retrieved from the database. The multiple archived coil sensitivity maps are then each used to reconstruct a magnetic resonance image from the magnetic resonance data. The image from the set of imaging magnetic resonance images that has the least artifacts may then be selected. This for example could be performed by displaying the set of imaging magnetic resonance images on a display and receiving input or by using an automatic algorithm.

In another embodiment, the imaging magnetic resonance image is selected from the set of imaging magnetic resonance images by receiving input from a user interface.

In another embodiment, the imaging magnetic resonance image is selected from the set of imaging magnetic resonance images by receiving a selection from an artifact detection algorithm. The artifact detection algorithm may for example look for the repetition of identical image elements within the same image. This may for example be able to automatically identify folding of the imaging magnetic resonance image.

In another embodiment, the archived coil sensitivity map is descriptive of a coil sensitivity measured from the subject.

In another embodiment, the archived coil sensitivity map is descriptive of a coil sensitivity map or matrix not measured from the subject.

In another embodiment, each of the multiple coil elements comprises a fiducial marker. The magnetic resonance imaging system comprises a fiducial marker locator. The fiducial marker locator could be implemented in a variety of different ways. For example the magnetic resonance imaging system may incorporate one or more cameras which are used to identify the location of the fiducial marker locators. In other examples the fiducial marker locator could for example be a marker which is detected in the magnetic resonance data. A sample of a known material could for example be placed within each of the multiple coil elements. In other examples a tuned or resonant RF circuit could be located within each of the multiple coil elements which is then identified within the magnetic resonance data.

Execution of the machine executable instructions further causes the processor to determine one or more fiducial marker locations of the multiple coil elements by controlling the fiducial marker locator. The one or more selection criteria comprise the one or more fiducial marker locations. This embodiment may be beneficial because it may provide for a means of automatically determining the location of the multiple coil elements with respect to the subject and/or the region of the subject which is being imaged. This may allow for automatically selecting the archived coil sensitivity map from the database.

In another embodiment, execution of the machine-executable instructions further cause the processor to reconstruct an intermediate coil image for each of the multiple coil elements. During parallel imaging magnetic resonance protocols data is acquired from each of the multiple coil elements. This data from each of the multiple coil elements is typically under sampled. However, the data from a specific coil element can be reconstructed into an image. For example the intensity of the magnetic resonance data in a reconstructed image may be used to locate the coil element relative to the imaging zone. Execution of the machine executable instructions further causes the processor to determine a coil element location from each intermediate coil image. The one or more selection criteria comprise the coil element location for each of the multiple coil elements. This embodiment may be beneficial because it may provide for a means of using the imaging magnetic resonance data to automatically locate the position of the multiple coil elements. This may then be used to select an archived coil sensitivity map which has the multiple coil elements in a similar configuration to those being currently measured.

In another aspect, the invention provides for a computer program product comprising machine-executable instructions for a processor controlling a magnetic resonance imaging system. The magnetic resonance imaging system comprises a radio-frequency system comprising multiple coil elements for acquiring imaging magnetic resonance data from a subject. Execution of the machine-executable instructions cause the processor to control the magnetic resonance imaging system to acquire imaging magnetic resonance data using imaging pulse sequence commands. The imaging pulse sequence commands are configured for controlling the magnetic resonance imaging system to acquire the imaging magnetic resonance data according to an imaging parallel magnetic resonance imaging protocol. Execution of the machine-executable instructions further cause the processor to reconstruct an imaging magnetic resonance image from the imaging magnetic resonance data The imaging magnetic resonance image is reconstructed by maximizing consistency between the imaging magnetic resonance data, the imaging magnetic resonance image, and an imaging coil sensitivity map. Execution of the magnetic resonance imaging system further cause the processor to store the imaging coil sensitivity map in the memory.

In another aspect, the invention provides for a method of operating a magnetic resonance imaging system. The magnetic resonance imaging system comprises a radio-frequency system comprising multiple coil elements for acquiring imaging magnetic resonance data from a subject. The method comprises controlling the magnetic resonance imaging system to acquire the imaging magnetic resonance data using imaging pulse sequence commands. The imaging pulse sequence commands are configured for controlling the magnetic resonance imaging system to acquire the imaging magnetic resonance data according to an imaging parallel magnetic resonance imaging protocol. The method further comprises reconstructing an imaging magnetic resonance image from the imaging magnetic resonance data according to the imaging parallel magnetic resonance imaging protocol. The imaging magnetic resonance image is reconstructed by maximizing consistency between the imaging magnetic resonance data, the imaging magnetic resonance image, and an imaging coil sensitivity map. Execution of the magnetic resonance imaging system further cause the processor to store the imaging coil sensitivity map in the memory.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage may be any volatile or non-volatile computer-readable storage medium.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the C programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, bluetooth connection, wireless local area network connection, TCP/IP connection, ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) display, Electroluminescent display (ELD), Plasma display panel (PDP), Liquid crystal display (LCD), Organic light-emitting diode display (OLED), a projector, and Head-mounted display.

Magnetic Resonance (MR) data is defined herein as being the recorded measurements of radio frequency signals emitted by atomic spins using the antenna of a magnetic resonance apparatus during a magnetic resonance imaging scan. Magnetic resonance data is an example of medical imaging data. A Magnetic Resonance (MR) image or magnetic resonance image data is defined herein as being the reconstructed two or three dimensional visualization of anatomic data contained within the magnetic resonance data.

### BRIEF DESCRIPTION OF THE DRAWINGS

In the following preferred embodiments of the invention will be described, by way of example only, and with reference to the drawings in which:
Fig. 1 illustrates an example of a magnetic resonance imaging system;
Fig. 2 shows a flow chart that illustrates a method of operating the magnetic resonance imaging system of Fig. 1;
Fig. 3 illustrates a further example of a magnetic resonance imaging system;
Fig. 4 illustrates a further example of a magnetic resonance imaging system; and
Fig. 5 shows a flow chart that illustrates a method of operating the magnetic resonance imaging system of Fig. 4.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

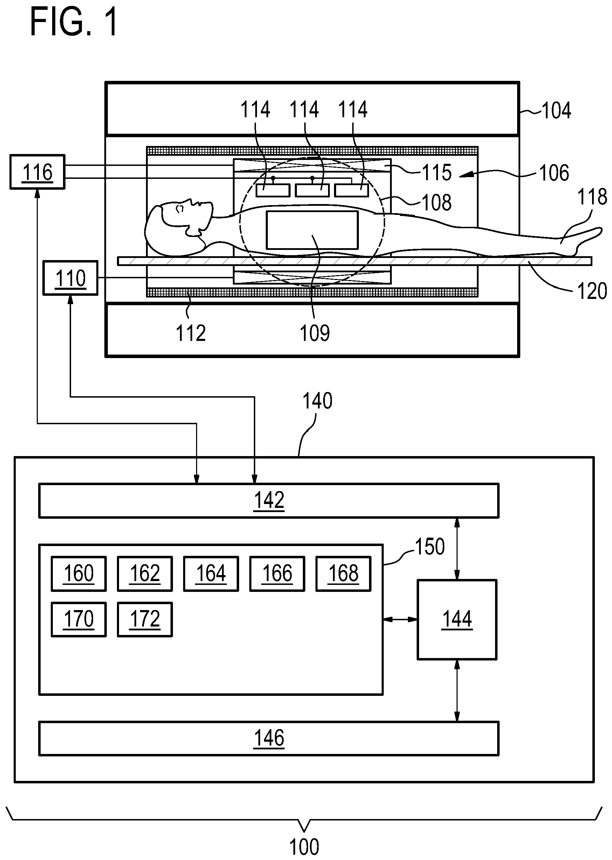

Fig. 1 illustrates an example of a magnetic resonance imaging system 100. The magnetic resonance imaging system 100 comprises a main magnet 104, which may be referred to as the magnet. The magnet 104 is a superconducting cylindrical type magnet 104 with a bore 106 through it. The use of different types of magnets is also possible. Inside the cryostat of the cylindrical magnet, there is a collection of superconducting coils. Within the bore 106 of the cylindrical magnet 104 there is an imaging zone 108 where the magnetic field is strong and uniform enough to perform magnetic resonance imaging.

Within the bore 106 of the magnet there is also a set of magnetic field gradient coils 110 which is used for acquisition of magnetic resonance data to spatially encode magnetic spins within the imaging zone 108 of the magnet 104. The magnetic field gradient coils 110 are connected to a magnetic field gradient coil power supply 112. The magnetic field gradient coils 110 are intended to be representative. Typically, magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 are multiple coil elements 114 that each function as radio-frequency antennas for manipulating the orientation of magnetic spins within the imaging zone 108 and for receiving radio transmissions from spins also within the imaging zone 108. The radio frequency coils may also be referred to as a radio frequency antennas or as antennas. The multiple coil elements may also be referred to as antenna elements. The radio frequency antennas may also be referred to as channels. The multiple coil elements 114 are connected to a radio frequency transceiver 116. The multiple coil elements 114 and radio frequency transceiver 116 may have separate transmitters and receivers for each the multiple coil elements 114. The coil elements 114 and the transceiver 116 form a radio-frequency system.

The coil elements 114 may be used to acquire magnetic resonance data separately. The coil elements 114 may therefore be used for a parallel imaging magnetic resonance technique. An optional body coil 115 is also shown. The body coil 115 would be useful in the parallel imaging technique as it could take acquired data at the same time as the individual coil elements 114 and be used for calculating a set of coil sensitivities.

The magnetic resonance data may be acquired from within the imaging zone 108. The location of a region of interest 109 is visible within the imaging zone 108.

It can be seen that different coil elements 114 are different distances from different regions of the region of interest 109. Different coil elements 114 will therefore be more or less sensitive to various portions of the imaging zone 109.

Within the bore 106 of the magnet 104 there is a subject support 120 which supports the subject in the the imaging zone 108.

The transceiver 116 and the gradient controller 130 are shown as being connected to a hardware interface 142 of a computer system 140. The computer system further comprises a processor 144 that is in communication with the hardware system 142, memory 150, and a user interface 146. The memory 150 may be any combination of memory which is accessible to the processor 144. This may include such things as main memory, cached memory, and also non-volatile memory such as flash RAM, hard drives, or other storage devices. In some examples the memory 150 may be considered to be a non-transitory computer-readable medium.

Within the memory 150 are located machine-executable instructions 160. The machine-executable instructions 160 enable the processor to control the operation and function of the magnetic resonance imaging system 100 via the hardware interface 142. The memory 150 is further shown as containing an imaging coil sensitivity map 162. The imaging coil sensitivity map 162 is descriptive of the coil sensitivity of the coil elements 114. In some examples the coil sensitivity map could also contain coil sensitivity data for virtual coil elements. The coil sensitivity map data for the physical coil elements 114 can be combined, either in hardware or in software, to reduce data size and/or processing demand. Where such combination is applied, the resulting coil sensitivity maps could reflect the combination algorithm in its metadata.

The computer memory 150 is further shown as containing imaging pulse sequence commands 164. The imaging pulse sequence commands are for controlling the magnetic resonance imaging system 100 to acquire imaging magnetic resonance data 166 according to an imaging parallel magnetic resonance imaging protocol.

The memory 150 is further shown as containing imaging magnetic resonance data 166 that has been acquired using the imaging pulse sequence commands 164. Pulse sequence commands as used herein encompass either instructions or data which can be converted into instructions for controlling the magnetic resonance imaging system for acquiring magnetic resonance data. The memory 150 is further shown as containing imaging magnetic resonance image 168 that has been reconstructed using the imaging magnetic resonance data 166 and the imaging coil sensitivity map 162. The memory is further shown as optionally containing further magnetic resonance data 170 that was acquired using further pulse sequence commands 172 to control the magnetic resonance imaging system.

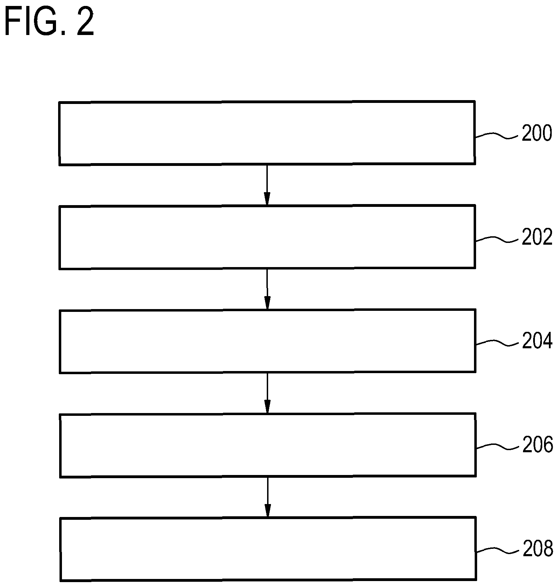

Fig. 2 shows a flowchart which illustrates a method of operating the magnetic resonance imaging system 100 of Fig. 1. First in step 200 the magnetic resonance imaging system 100 is controlled with the imaging pulse sequence commands 164 to acquire the imaging magnetic resonance data 166. Next in step 202 the imaging magnetic resonance image 168 is reconstructed from the imaging magnetic resonance data 166 according to the imaging parallel magnetic resonance imaging protocol. In step 202 the imaging magnetic resonance image 168 is reconstructed by iteratively maximizing consistency between the imaging magnetic resonance data 166, the imaging magnetic resonance image 168, and an imaging coil sensitivity map 162. In step 204, the imaging coil sensitivity map 162 is stored in the memory 150 for future use.

Fig. 2 also shows optional step 206, where the magnetic resonance imaging system 100 is controlled by the processor 144 to acquire further magnetic resonance data 170 using further pulse sequence commands 172 according to a further parallel magnetic resonance imaging protocol. In optional step 208, the processor reconstructs a further magnetic resonance image from the further magnetic resonance data using the imaging coil sensitivity map 162 according to the further parallel magnetic resonance imaging protocol.

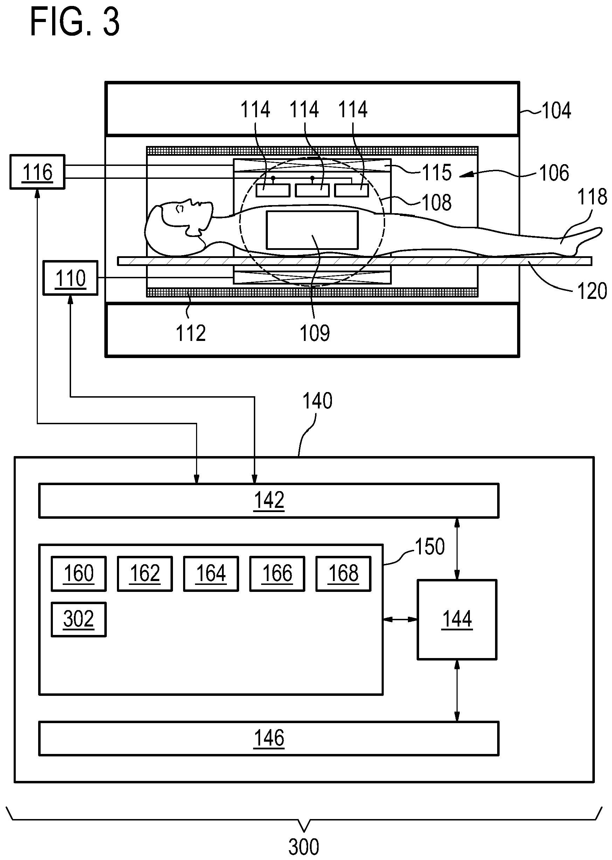

As a concrete example using the Magnetic resonance imaging system of Fig. 3, the further pulse sequence commands could be an implementation of a GRAPPA or GRAPPA type magnetic resonance imaging protocol. A subject 118 is placed into the magnetic resonance imaging system and imaged using the GRAPPA protocol. Normally, the imaging coil sensitivity map 162 that is determined during the GRAPPA reconstruction of the imaging magnetic resonance image 168 is discarded. According to a preferred embodiment, the imaging coil sensitivity map is stored in the memory 150.

Fig. 3 shows a further example of a magnetic resonance imaging system 300. The magnetic resonance imaging system 300 depicted in Fig. 3 is similar to the magnetic resonance imaging system 100 depicted in Fig. 1. It can be seen in Fig. 3 that the memory 150 is further shown as containing an archived coil sensitivity map 302 that is descriptive of coil sensitivities of the multiple coil elements 114. The imaging coil sensitivity map may be derived from the archived coil sensitivity map. For example the imaging parallel magnetic resonance imaging protocol may be an auto calibrating parallel imaging protocol such as a GRAPPA or eSPIRiT magnetic resonance imaging protocol. The archived coil sensitivity map may be used to start or seed the iterative process of reconstructing the imaging magnetic resonance image 168.

In other examples, the imaging parallel magnetic resonance imaging protocol could be a SENSE or SENSE type magnetic resonance imaging protocol. Such a situation could be particularly beneficial when the GRAPPA protocol is performed directly before imaging according to one or more SENSE protocol. The position of the subject and the relationship between the multiple coil elements may still be the same during the SENSE protocol and the GRAPPA protocol.

Another advantage to such a calibration scheme for the SENSE protocol is that the determination of the coil sensitivities can also be determined after the acquisition of the imaging magnetic resonance data has been acquired. This means that the quality of any images reconstructed according to the SENSE protocol using the imaging coil sensitivity map 162 can be controlled or checked for quality control. For example, if the archived coil sensitivity map 302 is incorrect it may result in folding artifacts in the resulting image. A human operator or even an automatic algorithm can detect these folding artifacts. If artifacts, or too many artifacts are detected, the magnetic resonance imaging system can be controlled to reacquire coil sensitivity map and then apply the SENSE protocol image reconstruction using the newly acquired sensitivity map. This is true if the archived coil sensitivity map was just acquired using a GRAPPA protocol or even if the archived coil sensitivity map was selected and retrieved from a database.

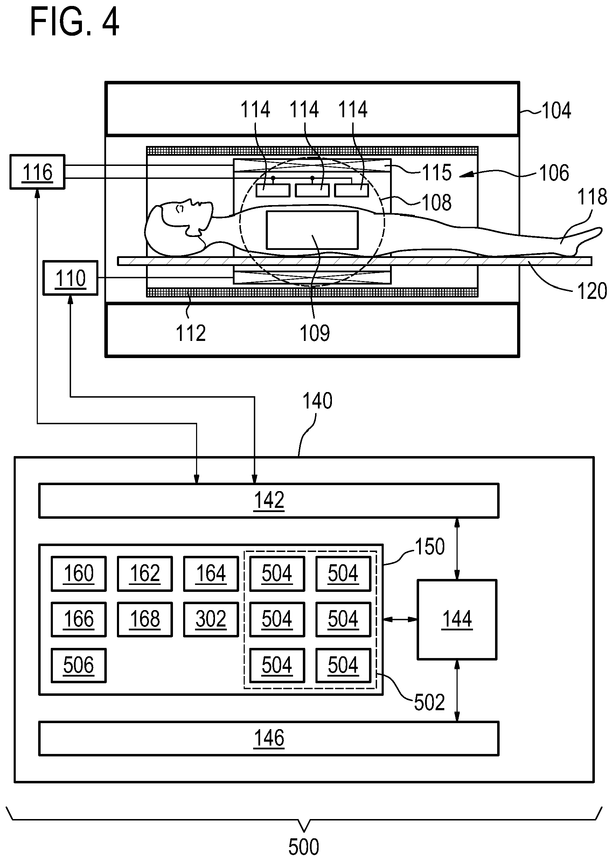

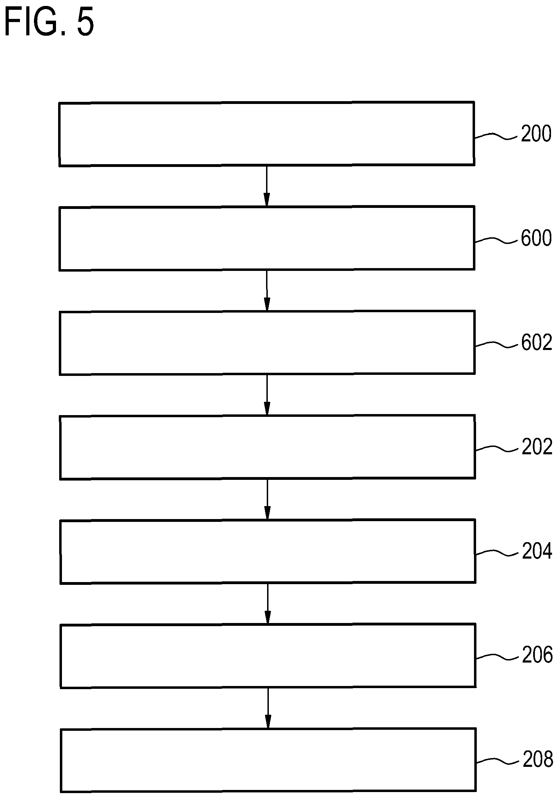

Fig. 4 illustrates a further example of a magnetic resonance imaging system 500. The magnetic resonance imaging system 500 of Fig. 5 is similar to the magnetic resonance imaging system 300 of Fig. 3.

The memory 150 is shown as further containing an implementation of a database 502. The database has multiple coil sensitivity map entries 504. Each of the coil sensitivity maps 504 also contains meta data descriptive of each of the coil sensitivity maps 504. The memory 150 is further shown as containing a number of selection criteria 506 which is descriptive of the acquisition of the imaging magnetic resonance data 166. In this example the selection criteria 506 was used to query the database 502. This resulted in the archived coil sensitivity map 162 being retrieved from the database 502.

The database with the multiple coil sensitivity map entries could be constructed in a variety of different ways. Whenever an explicit coil sensitivity map is measured it could be stored in the database 502 along with its associated meta data. After a period of time a larger and larger collection of coil sensitivities could be chosen from, which would increase the likelihood that a matching coil sensitivity map would be found that could be used to properly reconstruct the imaging magnetic resonance image 168. The database could also be added to when an auto calibrating parallel imaging protocol is performed. Instead of discarding the data from the calculation, the imaging coil sensitivity map 162 could be stored in the database along with metadata descriptive of the conditions under which the coil sensitivity map was acquired.

One potential difficulty is that there may not be a coil sensitivity map in the database that perfectly matches the current imaging conditions for when the imaging magnetic resonance data is acquired. There are several strategies that could assist in this situation. One technique would be to perform a cluster analysis of the metadata and then to retrieve the coil sensitivities cluster around the metadata for the imaging magnetic resonance data and then to make multiple image reconstructions with different coil sensitivity maps retrieved from the database 502. Each of the multiple image reconstructions could be examined for image artifacts, such as image folding. The image with the fewest artifacts could be selected. Alternatively, or in conjunction with the example a failure to reconstruct an acceptable imaging magnetic resonance image could trigger the acquisition of a new coil sensitivity map.

The use of the database 502 may be beneficial because it may enable the use of coil sensitivity maps from previous examinations or possibly even from coil sensitivity maps acquired from different subjects. The metadata can contain information which would allow the grouping of coil sensitivity maps acquired for similar examination types, patient positioning, and antenna locations.

Fig. 5 shows a flowchart which illustrates an example of a method of operating the magnetic resonance imaging system 500 of Fig. 4. The method illustrated in Fig. 5 is similar to the method illustrated in Fig. 2. The method of Fig. 5 starts with method step 200 of Fig. 2. Next in step 600 the one or more selection criteria 506 are received which are descriptive of the acquisition of the imaging magnetic resonance data 166. Next in step 602 the database 502 is queried using the selection criteria 506 to retrieve the archived coil sensitivity map 302. The database 502 is configured for selecting the archived coil sensitivity map 302 by comparing the selection criteria to the meta data which is part of each of the coil sensitivity map entries 504. In different examples this may be performed differently. In one example, the database may try to match the meta data exactly. In other examples the database may be configured for selecting the coil sensitivity map 504 or maps which closest resemble the selection criteria 506. For example, a neural network or clustering algorithm could be used for performing this. After step 602 is performed the method proceeds to step 202 as is depicted and described in Fig. 2.

In examples, the efficiency of Parallel Imaging may be maximized if the coil sensitivity calibration data is available from a prior scan. Image Quality can be optimized, however, by applying so called auto-calibration approaches, where the k-space convolution kernel is determined by oversampling the central part of k-space during the actual scan. This is not readily compatible with several types of k-space coverage, specifically EPI.

Auto calibration provides sufficient reconstruction approaches in the case of fold over in the image, and it supports improved resilience against gross motion that would occur between a pre-scan for calibration and the actual scan. The auto calibration data is part of the actual sequence and its sensitivity maps are only available as volatile data during the reconstruction.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

100 magnetic resonance system
104 main magnet
106 bore of magnet
108 imaging zone
109 region of interest
110 magnetic field gradient coils
112 gradient coil power supply
114 coil element
115 body coil
116 transceiver
118 subject
120 subject support
124 phase encoding direction
140 computer system
142 hardware interface
144 processor
146 user interface
150 computer memory
160 machine executable instructions
162 imaging coil sensitivity map
164 imaging pulse sequence commands
166 imaging magnetic resonance data
168 imaging magnetic resonance image
170 further magnetic resonance data
172 further pulse sequence commands
200 control the magnetic resonance imaging system to acquire the imaging magnetic resonance data using the imaging pulse sequence commands
202 reconstruct an imaging magnetic resonance image from the imaging magnetic resonance data according to the imaging parallel magnetic resonance imaging protocol
204 store the imaging coil sensitivity map in the memory
206 control the magnetic resonance imaging system to acquire further magnetic resonance data using further pulse sequence commands according to a further parallel magnetic resonance imaging protocol
208 reconstruct a further magnetic resonance image from the further magnetic resonance data using the archived coil sensitivity map according to the further parallel magnetic resonance imaging protocol
300 magnetic resonance imaging system
302 archived coil sensitivity map
500 magnetic resonance imaging system
502 database
504 coil sensitivity map
506 selection criteria
600 receive one or more selection criteria descriptive of the acquisition of the imaging magnetic resonance data
602 query the database with the one or more selection criteria to retrieve the archived coil sensitivity map

## Claims

1. A magnetic resonance imaging system (100, 300, 500) comprising:
a radio-frequency system (114, 116) comprising multiple coil elements (114) for acquiring imaging magnetic resonance data (166) from a subject (118);
a memory (150) for storing machine executable instructions (160), wherein the memory further stores imaging pulse sequence commands (164), wherein the imaging pulse sequence commands are configured for controlling the magnetic resonance imaging system to acquire the imaging magnetic resonance data according to a chosen parallel magnetic resonance imaging protocol,
a processor (144) for controlling the magnetic resonance imaging system,
wherein execution of the machine executable instructions causes the processor to:
control (200) the magnetic resonance imaging system to acquire the imaging magnetic resonance data using the imaging pulse sequence commands; and
reconstruct (202) an imaging magnetic resonance image (168) from the imaging magnetic resonance data according to the chosen parallel magnetic resonance imaging protocol, wherein the imaging magnetic resonance image is reconstructed by maximizing consistency between the imaging magnetic resonance data, the imaging magnetic resonance image, and an imaging coil sensitivity map; and
store (204) the imaging coil sensitivity map in the memory.

2. The magnetic resonance imaging system of claim 1, wherein execution of the machine executable instructions further cause the processor to:
control (206) the magnetic resonance imaging system to acquire further magnetic resonance data using further pulse sequence commands according to a further parallel magnetic resonance imaging protocol; and
reconstruct (208) a further magnetic resonance image from the further magnetic resonance data using the imaging coil sensitivity map according to the chosen parallel magnetic resonance imaging protocol.

3. The magnetic resonance imaging system of claims 1 or 2, wherein the imaging magnetic resonance image is reconstructed using a fast channel combination method that generates coil sensitivity data for virtual coil elements, and wherein execution of the machine executable instructions causes the processor to store the coil sensitivity data for the virtual coil elements and metadata descriptive of inclusion of the coil sensitivity data in the imaging coil sensitivity map in the memory.

4. The magnetic resonance imaging system of any one of the preceding claims, wherein the chosen parallel imaging magnetic resonance imaging protocol is an auto calibration parallel imaging protocol.

5. The magnetic resonance imaging system of claim 1, 2, or 3, wherein the memory further contains calibration pulse sequence commands for acquiring coil sensitivity magnetic resonance data according to a coil sensitivity calibration magnetic resonance imaging protocol, wherein execution of the machine executable instructions further causes the processor to:
control the magnetic resonance imaging system with the calibration pulse sequence commands to acquire the coil sensitivity magnetic resonance data; and
reconstruct a measured coil sensitivity map from the coil sensitivity magnetic resonance data, wherein the imaging coil sensitivity map is derived from the measured coil sensitivity map.

6. The magnetic resonance imaging system of claim 1, 2, or 3, wherein the memory further contains an archived coil sensitivity map (302) descriptive of coil sensitivities of the multiple coil elements, wherein the imaging coil sensitivity map is derived from the archived coil sensitivity map.

7. The magnetic resonance imaging system of claim 6, wherein the memory further contains a database (502), wherein the database comprises entries (504) for multiple coil sensitivity maps, wherein the database comprises a metadata entry for each of the multiple coil sensitivity maps, wherein the database is configured for searching the metadata entry for each of the multiple coil sensitivity maps, wherein execution of the machine executable instructions further causes the processor to:
receive (600) one or more selection criteria (506) descriptive of the acquisition of the imaging magnetic resonance data; and
query (602) the database with the one or more selection criteria to retrieve the archived coil sensitivity map, wherein the database is configured for selecting the archived coil sensitivity map by comparing the selection criteria to the metadata .

8. The magnetic resonance imaging system of claim 7, wherein the metadata and/or the one or more selection criteria comprise any one of the following: a field of view, an off center condition, a subject support position, a coil identifier or serial number, a radio frequency system identifier or serial number, a subject weight, a subject age, a subject volume, a selected anatomical profile, a slice selection, a slice orientation, a magnetic resonance imaging scan protocol type, one or more antenna element orientations or positions, a magnetic resonance imaging system identifier or serial number, a subject identifier, a subject name, and combinations thereof.

9. The magnetic resonance imaging system of claim 7 or 8, wherein execution of the machine executable instructions further causes the processor to:
retrieve multiple archived coil sensitivity maps from the database, wherein the archived coil sensitivity maps comprise the archived coil sensitivity map;
reconstruct a set of imaging magnetic resonance images from the imaging magnetic resonance data using each of the archived coil sensitivity maps according to the imaging parallel magnetic resonance imaging protocol; and
select the imaging magnetic resonance image from the set of imaging magnetic resonance images.

10. The magnetic resonance imaging system of claim 8, wherein the imaging magnetic resonance image is selected from the set of imaging magnetic resonance images using any one of the following: input received from a user interface (146), a selection from an artifact detection algorithm, and combinations thereof.

11. The magnetic resonance imaging system of any one of claims 5 through 9, wherein any one of the following:
the archived coil sensitivity map is descriptive of a coil sensitivity map measured from the subject and
the archived coil sensitivity map is descriptive of a coil sensitivity map not measured from the subject.

12. The magnetic resonance imaging system of any one of claims 7 through 11, wherein any one of the following:
wherein each of the multiple coil elements comprises a fiducial marker, wherein the magnetic resonance imaging system comprises a fiducial maker locator, wherein execution of the machine executable instructors causes the processor to determine one or more fiducial marker locations of the multiple coil elements by controlling the fiducial marker locator, wherein the the one or more selection criteria comprise the one or more fiducial marker locations; and
wherein execution of the machine executable instructions further cause the processor to: reconstruct an intermediate coil image for each of the multiple coil elements; and determine a coil element location from each intermediate coil image, wherein the the one or more selection criteria comprise the coil element location for each of the multiple coil elements.

13. A computer program product comprising machine executable instructions (160) for a processor (144) controlling a magnetic resonance imaging system (100, 300, 500), wherein the magnetic resonance imaging system comprises a radio-frequency system (114, 116) comprising multiple coil elements (114) for acquiring imaging magnetic resonance data (166) from a subject (118), wherein execution of the machine executable instructions causes the processor to:
control (200) the magnetic resonance imaging system to acquire the imaging magnetic resonance data using imaging pulse sequence commands (164), wherein the imaging pulse sequence commands are configured for controlling the magnetic resonance imaging system to acquire the imaging magnetic resonance data according to a chosen parallel magnetic resonance imaging protocol; and
reconstruct (202) an imaging magnetic resonance image (168) from the imaging magnetic resonance data according to the chosen parallel magnetic resonance imaging protocol, wherein the imaging magnetic resonance image is reconstructed by maximizing consistency between the imaging magnetic resonance data, the imaging magnetic resonance image, and an imaging coil sensitivity map; and
store (202) the imaging coil sensitivity map in the memory.

14. A method of operating a magnetic resonance imaging system (100, 300, 500), wherein the magnetic resonance imaging system comprises a radio-frequency system (114, 116) comprising multiple coil elements (114) for acquiring imaging magnetic resonance data (166) from a subject (118), wherein the method comprises:
controlling (200) the magnetic resonance imaging system to acquire the imaging magnetic resonance data using imaging pulse sequence commands (164), wherein the imaging pulse sequence commands are configured for controlling the magnetic resonance imaging system to acquire the imaging magnetic resonance data according to a chosen parallel magnetic resonance imaging protocol; and
reconstructing (202) an imaging magnetic resonance image (168) from the imaging magnetic resonance data according to the chosen parallel magnetic resonance imaging protocol, wherein the imaging magnetic resonance image is reconstructed by maximizing consistency between the imaging magnetic resonance data, the imaging magnetic resonance image, and an imaging coil sensitivity map; and
store (202) the imaging coil sensitivity map in the memory.
